# EUROPEAN PATENT SPECIFICATION

(11) **EP 4 082 590 B1**
(45) Date of publication and mention of the grant of the patent: **01.05.2024**
(21) Application number: 20906077.1
(22) Date of filing: 16.12.2020
(51) Int. Cl.: A61M 16/00, A61M 16/06, F04D 25/08

(54) **VENTILATOR**
LÜFTER
VENTILATEUR

(30) Priority: 27.12.2019 CN 201911381352
(43) Date of publication of application: 02.11.2022
(73) Proprietor: BMC Medical Co., Ltd., Shijingshan Beijing 100041 (CN)
(72) Inventor: LIU, Lijun, Beijing 100041 (CN); ZHUANG, Zhi, Beijing 100041 (CN)
(74) Representative: Grünecker Patent- und Rechtsanwälte PartG mbB
(86) International application number: PCT/CN2020/136746
(87) International publication number: WO 2021/129479

(56) References cited:
- WO-A1-2015/048849
- WO-A2-2015/004418
- CN-A- 101 424 278
- CN-A- 105 617 497
- CN-A- 111 110 968
- CN-U- 201 802 647
- CN-U- 201 902 349
- CN-U- 202 381 413
- CN-Y- 2 629 730
- GB-A- 2 475 425
- US-B2- 10 124 140

## Description

The present application claims the priority of the Chinese patent application filed on December 27th, 2019 before the Chinese Patent Office with the application number of 201911381352.5 and the title of "VENTILATOR".

### TECHNICAL FIELD

The present disclosure relates to a field of ventilatory treatment apparatus and, more particularly, relates to a ventilator.

### BACKGROUND

An existing ventilator usually includes a host, a ventilation tube and a breathing mask, the ventilation tube is connected with the host and the breathing mask to conduct breathing gas generated by the host to the breathing mask for a patient to inhale. The host usually includes a noise-reducing apparatus and a blower disposed in the noise-reducing apparatus. During usage, air is sequentially inhaled into an air inlet end of the host, an air inlet end of the noise-reducing apparatus and the blower by the operation of the blower, and then high-pressure gas is sequentially discharged from an air outlet end of the noise-reducing apparatus and an air outlet end of the host through the blower, for the patient to inhale.

However, the existing ventilator also has the following problems:
(1) No matter the blower is a centrifugal fan or an axial fan, they all utilize a rotor to drive an impeller and an impeller blade thereof to directly discharge high-pressure gas from the air outlet of the blower. When the high-pressure gas passes through the air outlet, it is basically in a turbulent state. Fluid in the turbulent state is mixed with each other and moves disorderly, which may lead to an energy loss, an airflow fluctuation and an abnormal noise and so forth.
(2) The blower needs to be installed in the noise-reducing apparatus by using a buffer structure, in addition, a high pressure area and a low pressure area in the noise-reducing apparatus needs to be separated and sealed. The air inlet end of the noise-reducing apparatus needs to be connected and sealed with the air inlet end of the host, the air outlet end of the noise-reducing apparatus needs to be connected and sealed with the air outlet end of the host. Therefore, the host has a complex structure with many parts, there are more places needs to be sealed, and there is a high leakage risk.
(3) If generating a higher pressure and/or flow is required, the blower needs to adjust speed of the rotor at equal ratio to drive the impeller blades to rotate at a higher speed, which may lead to a rotor heating, an energy loss, a shortened life and a sudden increase in noise and so forth. Document WO 2015/004418 A2 is directed to a fan assembly. The fan assembly comprises a stand, having an air inlet in the form of a plurality of apertures formed in an outer casing of the stand, and through which a primary air flow is drawn into the stand from the external environment. An annular nozzle, having an air outlet for emitting the primary air flow from the fan assembly is connected to the upper end of the stand. The nozzle comprises an outer wall extending about an annular inner wall. The inner wall defines a bore, or opening of the nozzle. Document US 10 124 140 B2 describes an atmosphere conditioning device. An air motivator may be a bladeless fan that blows air from a ring with no external blades. The air is drawn in through an air intake by a fan in the case and then directed up into the ring to exit through an aperture or crack all around the ring and passes over a shape like that of an aircraft wing.

### SUMMARY

An objective of the present disclosure is to provide a new type of ventilator, to solve at least one of the problems as mentioned above.

The invention is described in the claims. The invention provides a ventilator according to claim 1. Preferred embodiments are described in the dependent claims. The embodiments which do not fall within the scope of the claims are to be interpreted as examples useful for a better understanding of the invention. In order to implement the objective as mentioned above, the present disclosure provides a ventilator, the ventilator includes a ventilation body, the ventilation body includes a ventilation cavity and an air inlet end and an air outlet end communicating with the ventilation cavity, the ventilation body further includes an annular shell configured to form the ventilation cavity, the annular shell is formed with an annular cavity inside, an air inlet and an air outlet communicating with the annular cavity are disposed on the annular shell, the air outlet is communicated with the annular cavity and the ventilation cavity, the air outlet has a slit shape extending along a circumferential direction of the annular shell and is disposed to be capable to guide gas flows out towards the air outlet end.

Optionally, the air outlet is disposed close to the air inlet end. Which leads to a greater negative pressure on one side of the air inlet end of the ventilation body, to further increase a ventilation volume.

Optionally, one side edge of the air outlet close to the air inlet end is disposed to bend and extend towards the other side edge of the air outlet, and the side edge is closer to a central axis of the annular shell in a radial direction of the annular shell compared with the other side edge. In this way, gas flowing out of the air outlet may enter the ventilation cavity and flow towards the air outlet end under guidance of the side edge of the air outlet, to achieve the functions of the air outlet as mentioned above.

Optionally, the other side edge of the air outlet is disposed to bend and extend in a direction away from the side edge, which makes air flows smoother.

Optionally, a minimum spacing between the side edge of the air outlet and the other side edge is less than 2mm, which further improves the pressure of gas flowing out of the air outlet.

Optionally, a width of the annular cavity gradually decreases along a direction from the air inlet end to the air outlet end. Through the above disposing, gas entering the annular cavity is squeezed to a direction of the air outlet, thus further to improve the air pressure of the air outlet.

Optionally, a diameter of the ventilation cavity gradually increases along a direction from the air inlet end to the air outlet end. Thereby air flow through the air outlet end may be increased.

Optionally, the ventilator includes a base and a power motor, the base is formed with an cavity inside, an air inlet hole and an air outlet hole communicating with the cavity are disposed on the base, the power motor is disposed inside the cavity, the annular shell is installed on the base and the air inlet is made to be sealed and communicated with the air outlet hole. Through the above disposing, during usage, the power motor may inhale outside gas into the cavity through the air inlet hole, and the gas flows into the annular cavity through the air outlet hole and the air inlet. Wherein, the gas may be accelerated into the annular cavity through a cyclone accelerator of the power motor, thus an exhausting speed of the air outlet of the ventilation body may be increased several times, to further increase a negative pressure of one side of the air inlet end of the ventilation body, to make the ventilation volume to increase several times.

Optionally, the ventilator includes a buffer part, the buffer part is located inside the cavity and disposed to surround the power motor, which reduces running noise of the power motor.

Optionally, the base includes a bottom wall and a surrounding wall configured to form the cavity, a top opening of the cavity forms as the air outlet hole, and the air inlet hole is disposed on the surrounding wall.

Optionally, the power motor is formed to have a column shape, the buffer part is formed to have a cylinder shape and clamped between the power motor and the surrounding wall, the buffer part is air-permeable. The buffer plays a role in buffering and reducing noise, and achieves the effect of ventilation.

Optionally, the ventilator includes a main control component, the main control component is disposed in the cavity and configured to control the operation of the power motor, and is communicatively connected with a control terminal, which facilitates interactions at any moment between doctors and patients, to achieve intelligent medical treatment.

Optionally, the ventilator includes a heat-humidity exchanger, the heat-humidity exchanger is installed at the air outlet end. Therefore, the ventilator has a humidifying function.

Optionally, the ventilator includes a filter apparatus, the filter apparatus is disposed at the air inlet end. Therefore, gas entering the ventilation cavity is filtered to ensure safety of the inhaled gas.

The ventilator includes a breathing mask provided with a breathing cavity, the ventilation body is connected with the breathing mask and the air outlet end is made to be sealed and communicating with the breathing cavity.

The ventilator of the present disclosure by using the ventilation body of the technical solutions as mentioned above, during usage, after entering the annular gas cavity through the air inlet, the gas may form a vortex in the annular gas cavity and fill the annular gas cavity, and then flow out to the ventilation cavity through the air outlet, which may effectively increase the gas flow. Moreover, since the air outlet has a slit shape, the gas pressure can be greatly increased. In addition, under the guidance of the air outlet, the gas may flow towards the air outlet end after entering the ventilation cavity, to form a negative pressure on one side of the air inlet end of the ventilation body. Under the negative pressure, the gas of the side may enter the ventilation cavity through the air inlet end and flow towards the air outlet end, thus the ventilation volume of the ventilation body may be greatly increased. The ventilator of the present disclosure may increase the ventilation volume and the gas pressure through the ventilation body, without increasing the rotor speed by the blower as the existing ventilator, and the gas discharged from the air outlet may not form a turbulence, which may make gas to flow smoother and to reduce energy consumption.

Other features and advantages of the present disclosure may be described in details in the subsequent description of the embodiments.

The above description is merely a summary of the technical solutions of the present disclosure. In order to more clearly know the elements of the present disclosure to enable the implementation according to the contents of the description, and in order to make the above and other objectives, features and advantages of the present disclosure more apparent and understandable, the particular embodiments of the present disclosure are provided below.

### BRIEF DESCRIPTION OF THE DRAWINGS

In order to more clearly illustrate the technical solutions of the embodiments of the present disclosure or the prior art, the figures that are required to describe the embodiments or the prior art will be briefly introduced below. Apparently, the figures that are described below are embodiments of the present disclosure, and a person skilled in the art can obtain other figures according to these figures without paying creative work.

The attached figures are configured to provide further understanding of the present disclosure, and to be part of the specification and are used to explain the present disclosure with the detailed description of the embodiments, but it does not limit the present disclosure. In the attached figures:
FIG. 1 is a stereogram of an embodiment of a ventilator of the present disclosure;
FIG. 2 is a sectional view of FIG. 1;
FIG. 3 is another sectional view of FIG. 1;
FIG. 4 is a schematic diagram of an airflow direction when using the ventilator in FIG. 3; and
FIG. 5 is an explosion diagram of FIG. 3.

### Explanations of labels of the attached figures

10-ventilation body, 11-ventilation cavity, 12-air inlet end, 13-air outlet end, 14-annular shell, 15-annular cavity, 16-air inlet, 17-air outlet, 171-one side edge, 172-the other side edge, 20-base, 21-cavity, 22-air inlet hole, 23-air outlet hole, 24-bottom wall, 25-surrounding wall, 30-power motor, 40-buffer part.

### DETAILED DESCRIPTION OF THE EMBODIMENTS

In order to make the objects, the technical solutions and the advantages of the embodiments of the present disclosure clearer, the technical solutions of the embodiments of the present disclosure will be clearly and completely described below with reference to the drawings of the embodiments of the present disclosure. Apparently, the described embodiments are merely certain embodiments of the present disclosure, rather than all of the embodiments. All of the other embodiments that a person skilled in the art obtains on the basis of the embodiments of the present disclosure without paying creative work fall within the protection scope of the present disclosure.

The following is a detailed description of the specific implementation of the present disclosure in combination with the attached drawings. It should be understood that the specific implementation described herein are intended only to illustrate and explain the present disclosure and are not intended to limit the present disclosure.

In the present disclosure, in the absence of a statement to the contrary, locational terms such as "top, bottom" are usually used to refer to the locations shown in the attached figures. "Inside, outside" refer to the inside and the outside of each part relative to its own contour.

The present disclosure provides a ventilator, the ventilator includes a ventilation body 10, the ventilation 10 includes a ventilation cavity 11 and an air inlet end 12 and an air outlet end 13 communicating with the ventilation cavity 11, the ventilation body 10 further includes an annular shell 14 configured to form the ventilation cavity 11, inside the annular shell 14 is formed with an annular cavity 15, an air inlet 16 and an air outlet 17 communicating with the annular cavity 15 are disposed on the annular shell 14, the air outlet 17 is communicated with the annular cavity 15 and the ventilation cavity 11, the air outlet 17 has a slit shape extending along a circumferential direction of the annular shell 14 and is disposed to be capable to guide gas flows out towards the air outlet 13 end.

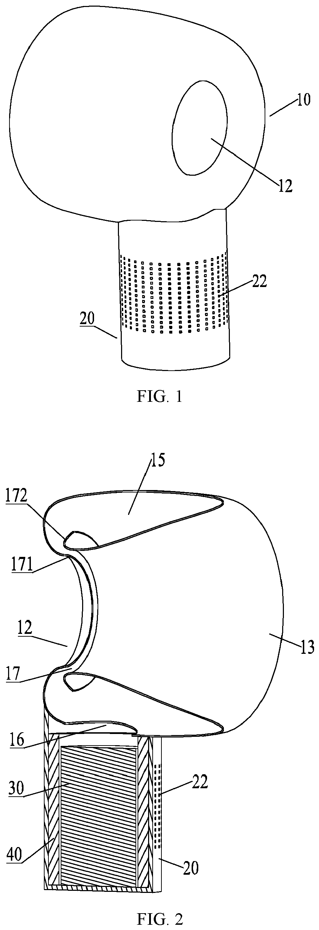

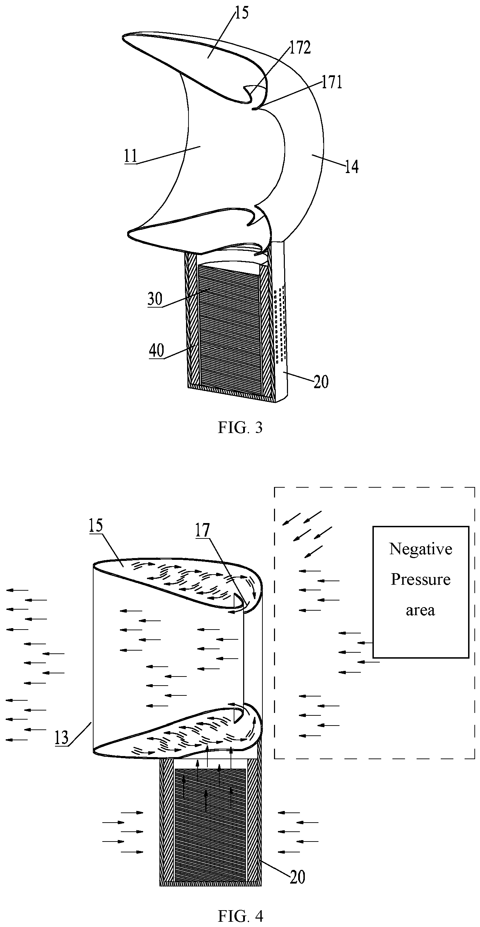

In the above, the annular shell 14 may include an inner surrounding wall and an outer surrounding wall configured to form the annular cavity 15, two ends of the inner surrounding wall and the outer surrounding wall are sealed and connected to form as an entirety, the ventilation cavity 11 is formed by the inner surrounding wall, the air outlet 17 is disposed on the inner surrounding wall and the air inlet 16 is disposed on the outer surrounding wall (referring to FIG. 2 and FIG. 3).

The ventilator of the present disclosure by using the ventilation body 10 of the technical solutions as mentioned above, during usage, after entering the annular gas cavity 15 through the air inlet 16, the gas may form a vortex in the annular gas cavity 15 and fills the annular gas cavity 15, and then flow out to the ventilation cavity 11 through the air outlet 17, which may effectively increase the gas flow. Moreover, since the air outlet 17 has a slit shape, the gas pressure may be greatly increased. In addition, under the guidance of the air outlet 17, the gas may flow towards the air outlet end 13 after entering the ventilation cavity 11, to form a negative pressure on one side of the air inlet end 12 of the ventilation body 10 (as referring to FIG. 4). Under the negative pressure, the gas of the side may enter inside the ventilation cavity 11 through the air inlet end 12 and flow towards the air outlet end 13, thus the ventilation volume of the ventilation body 10 may be greatly increased. The ventilator of the present disclosure may increase the ventilation volume and the gas pressure through the ventilation body 10, without increasing the rotor speed by the blower as the existing ventilator, and the gas discharged from the air outlet 17 may not form a turbulence, which may make gas to flow smoother and to reduce energy consumption.

In the present disclosure, in order to form a larger negative pressure on one side of the air inlet end 12 of the ventilation body 10, to further increase the ventilation volume, as referring to FIG. 2 and FIG. 3, the air outlet 17 is preferably disposed close to the air inlet end 12.

In the present disclosure, in order to achieve the guidance function of the air outlet 17 as mentioned above, as referring to FIG. 2 and FIG. 3, one side edge 171 of the air outlet 17 close to the air inlet end 12 is disposed to bend and extend towards the other side edge 172 of the air outlet 17, and the side edge 171 is closer to a central axis of the annular shell 14 in a radial direction of the annular shell 14 compared to the other side edge 172. In this way, gas flowing out through the air outlet 17 may enter the ventilation cavity 11 and flow towards the air outlet end 13 under the guidance of the side edge 171 of the air outlet 17.

Further, as referring to FIG. 2 and FIG. 3, the other side edge 172 of the air outlet 17 is disposed to bend and extend in a direction away from the side edge 171 to make gas to flow smoother. Wherein, the other side edge 172 may be disposed to extend towards the inside of the annular cavity 15 (as referring to FIG. 2 and FIG. 3), or it may be disposed to extend towards the inside of the ventilation cavity 11. Wherein, by disposing the other side edge 172 to extend towards the inside of the annular cavity 15, it is beneficial to gas entering from the air inlet 16 to fill inside of the annular cavity 15 and increase pressure when the gas flows out though the air outlet 17.

In the present disclosure, in order to further increase the pressure of the gas flowing out from the air outlet 17, as a preferable option, a minimum spacing between the side edge 171 and the other side edge 172 of the air outlet 17 is less than 2mm, such as 1mm.

In the present disclosure, as a preferable option, as referring to FIG. 3, a width of the annular cavity 15 (that is the radial spacing between the inner surrounding wall and the outer surrounding wall of the annular shell 14) gradually decreases along a direction from the air inlet end 12 to the air outlet end 13. Through the above disposing, gas entering the annular cavity 15 is squeezed to a direction of the air outlet 17, thus to further increase gas pressure of the air outlet 17. In addition, as a preferable option, a diameter of the ventilation cavity 11 gradually increases along a direction from the air inlet end 12 to the air outlet end 13, thus to increase the gas flow discharged through the air outlet end 13.

In the present disclosure, as shown in FIGs. 1-3, the ventilator may further include a base 20 and a power motor 30, the base 20 is formed with an cavity 21 inside, an air inlet hole 22 and an air outlet hole 23 communicating with the cavity 21 are disposed on the base 20, the power motor 30 is disposed inside the cavity 21, the annular shell 14 is installed on the base 20 and the air inlet 16 is made to be sealed and communicated with the air outlet hole 23. Through the above disposing, during usage, the power motor 30 may inhale outside gas into the cavity 21 through the air inlet hole 22, and the gas flows into the annular cavity 15 through the air outlet hole 23 and the air inlet 16. Wherein, the gas may be accelerated into the annular cavity 15 through a cyclone accelerator of the power motor, thus an exhausting speed of the air outlet 17 of the ventilation body 10 may be increased several times, to further increase a negative pressure of one side of the air inlet end 12 of the ventilation body 10, to make the ventilation volume to increase several times.

Wherein, in order to reduce the running noise of the power motor 30, the ventilator may further include a buffer part 40, the buffer part 40 is located inside of the cavity 21 and disposed to surround the power motor 30.

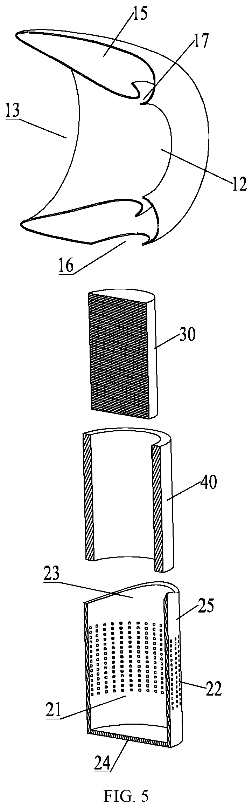

In the above, the base 20, the power motor 30 and the buffer part 40 may be provided with any suitable structures. According to an embodiment of the present disclosure, as shown in FIG. 3 and FIG. 5, the base 20 may include a bottom wall 24 and a surrounding wall 25 configured to form the cavity 21, a top opening of the cavity 21 is formed as the air outlet hole 23, and the air inlet hole 22 is disposed on the surrounding wall 25, the power motor 30 may be formed as a column shape, the buffer part 40 may be formed as a cylinder shape and clamped between the power motor 30 and the surrounding wall 25. In this case, the buffer part 40 is air-permeable, to facilitate gas entering from the air inlet hole 22 to penetrate the buffer part 40 to flow upwardly. Wherein, the buffer part 40 may be made of sponge, or the buffer part 40 may also be a double-layer structure formed by a silica-gel layer and a sponge layer stacked, wherein the silica-gel layer is disposed close to the power motor 30. In other words, under the condition that the buffer part 40 does not affect the gas entering from the air inlet hole 22 to flow upwardly (such as there is a space between the buffer part 40 and the surrounding wall 25), the buffer part 40 may be only made of elastic materials, to play a role in buffering noise; under the condition that the buffer part 40 affects the gas entering from the air inlet hole 22 to flow upwardly (such as there is no space between the buffer part 40 and the surrounding wall 25 shown in FIG. 3), the buffer part 40 may be made of elastic materials and breathable materials together, to achieve the effect of air permeability while buffering and reducing noise.

Wherein, a plurality of air inlet hole 22 may be disposed on the base 20, as shown in FIG. 1, the plurality of air inlet hole 22 may be interval disposed along a circumferential direction of the surrounding wall 25, and disposed in many rows along an axial direction of the surrounding wall 25.

In the present disclosure, the ventilator may further include a main control component, the main control component is disposed in the cavity 21 and configured to control the operation of the power motor 30, and is communicatively connected with a control terminal. Wherein, the control terminal may be an APP located at a PC terminal or a mobile terminal, the main control component may communicate with the APP through Bluetooth, infrared, WIFI and data lines and so forth, patients may achieve controlling the power motor 30 through operating the APP. In addition, the main control component may be disposed to be capable to detect data ,such as gas flow, gas pressure, gas temperature and humidity, blood-oxygen concentration and gas composition and so forth, and send these data to the APP, to facilitate interactions at any moment between doctors and patients, to achieve the intelligent medical treatment.

In addition, the ventilator of the present disclosure may also have a humidification function. For example, a heat-humidity exchanger may be disposed at the air outlet end 13 of the ventilation body 10. During usage, the heat-humidity exchanger may retain a part of the moisture and the heat in the patient's exhaled gas, and which may be returned to the respiratory tract as during the process that the patient inhales gas.

In the present disclosure, the ventilator may further include a filter apparatus, the filter apparatus is disposed at the air inlet end 12 of the ventilation body 10 in order to filter the gas entering the ventilation cavity 11 and ensure safety of the inhaled gas.

When the ventilator of the present disclosure is used, the ventilator also includes a breathing mask with a breathing cavity, the ventilation body 10 is connected with the breathing mask and the air outlet end 13 is sealed and communicated with the breathing cavity. Wherein, the ventilation body 10 may be directly installed on the breathing mask, or it may be connected to the breathing mask through a ventilation tube.

The ventilator of the present disclosure has the following advantages: a large ventilation volume, a large gas pressure, low energy consumption, a low air fluctuation, a low noise, fewer parts and components, a simple structure, a small volume, easy to carry, convenient production and maintenance and various ways to use.

The preferred embodiment of the present disclosure is described in detail above in combination with the attached drawings. However, the present disclosure is not limited to the specific details of the above embodiment. Within the scope of the technical idea of the present disclosure, a plurality of simple variations the technical solutions of the present disclosure can be carried out, and these simple variations fall within the scope of protection of the present claims.

It should also be noted that the specific technical features described in the above specific embodiments may be combined in any appropriate manner without contradiction. In order to avoid unnecessary repetition, the present disclosure does not specify the various possible combinations.

The device embodiments described above is only schematic, the units illustrated as separated parts may be or may not be separated physically, and the parts shown in unit may be or may not be a physical unit. That is, the parts may be located at one place or distributed in multiple network units. A skilled person in the art may select part or all modules therein to realize the objective of achieving the technical solution of the embodiment. Those of ordinary skill in the art can understand and implement it without creative effort.

The term "one embodiment", "an embodiment", or "one or more embodiments" herein means that the particular features, structures, or features described in combination with embodiments are included in at least one embodiment disclosed herein. Also, note that the examples of words "in an embodiment" here do not necessarily all refer to the same embodiment.

A great deal of detail is provided in the manual provided here. However, it is understood that this disclosed embodiment can be practiced without such specific details. In some instances, known methods, structures and techniques are not detailed so as not to obscure the understanding of this specification.

In a claim, no reference symbol between parentheses shall be constructed to restrict the claim. The word "include" does not exclude the existence of elements or steps not listed in the claim. The word "one" or "one" before a component does not preclude the existence of more than one such component. This exposure can be implemented with the help of hardware including several different components and with the help of properly programmed computers. In listing the unit claims of several devices, several of these devices can be embodied by the same hardware item. The use of the words first, second, and third does not indicate any order. These words can be interpreted as names.

Finally, it should be noted that the above embodiments are only used to illustrate, and not to limit, the disclosed technical solution; notwithstanding the detailed description of the present disclosure with reference to the foregoing embodiments, ordinary technical personnel in the field should understand that they can still modify the technical solutions recorded in the foregoing embodiments, or make equivalent substitutions to some of the technical features thereof; such modifications or substitutions shall not separate the essence of the corresponding technical solutions from the scope of the technical solutions of the appended claims.

## Claims

1. A ventilator, wherein, the ventilator comprises a ventilation body (10), the ventilation body (10) comprises a ventilation cavity (11) and an air inlet end (12) and an air outlet end (13) communicating with the ventilation cavity (11), the ventilation body (10) further comprises an annular shell (14) configured to form the ventilation cavity (11), the annular shell (14) is formed with an annular cavity (15) inside, an air inlet (16) and an air outlet (17) communicating with the annular cavity (15) are disposed on the annular shell (14), the air outlet (17) is communicated with the annular cavity (15) and the ventilation cavity (11), the air outlet (17) has a slit shape extending along a circumferential direction of the annular shell (14) and is disposed to be capable to guide gas flows out towards the air outlet end (13);
**characterized in that**
the ventilator comprises a breathing mask provided with a breathing cavity, the ventilation body (10) is connected with the breathing mask and the air outlet end (13) is made to be sealed and communicating with the breathing cavity.

2. The ventilator according to claim 1, wherein, the air outlet (17) is disposed close to the air inlet end (12); and/or
one side edge (171) of the air outlet (17) close to the air inlet end (12) is disposed to bend and extend towards the other side edge (172) of the air outlet (17), and the side edge (171) is closer to a central axis of the annular shell (14) in a radial direction of the annular shell (14) compared with the other side edge (172).

3. The ventilator according to claim 2, wherein,
the other side edge (172) of the air outlet (17) is disposed to bend and extend in a direction away from the side edge (171); and/or
a minimum spacing between the side edge (171) of the air outlet (17) and the other side edge (172) is less than 2mm.

4. The ventilator according to any one of claims 2-3, wherein,
a width of the annular cavity (15) gradually decreases along a direction from the air inlet end (12) to the air outlet end (13); and/or
a diameter of the ventilation cavity (11) gradually increases along a direction from the air inlet end (12) to the air outlet end (13).

5. The ventilator according to any one of claims 1-4, wherein the ventilator comprises a base (20) and a power motor (30), the base (20) is formed with a cavity (21) inside, an air inlet hole (22) and an air outlet hole (23) communicating with the cavity (21) are disposed on the base (20), the power motor (30) is disposed inside the cavity (21), the annular shell (14) is installed on the base (20) and the air inlet (16) is made to be sealed and communicating with the air outlet hole (23).

6. The ventilator according to claim 5, wherein,
the ventilator comprises a buffer part (40), the buffer part (40) is located inside the cavity (21) and disposed to surround the power motor (30); and/or
the base (20) comprises a bottom wall (24) and a surrounding wall (25) configured to form the cavity (21), a top opening of the cavity (21) forms the air outlet hole (23), and the air inlet hole (22) is disposed on the surrounding wall (25).

7. The ventilator according to claim 6, wherein, the power motor (30) is formed to have a column shape, the buffer part (40) is formed to have a cylinder shape and clamped between the power motor (30) and the surrounding wall (25), the buffer part (40) is air-permeable.

8. The ventilator according to any one of claims 5-7, wherein, the ventilator comprises a main control component, the main control component is disposed in the cavity (21) and configured to control the operation of the power motor, and is communicatively connected with a control terminal.

9. The ventilator according to any one of claims 1-8, wherein, the ventilator comprises a heat-humidity exchanger, the heat-humidity exchanger is installed at the air outlet end (13);
and/or
the ventilator comprises a filter apparatus, the filter apparatus is disposed at the air inlet end (12).

## Patentansprüche

1. Ventilator, wobei der Ventilator einen Ventilationskörper (10) umfasst, wobei der Ventilationskörper (10) einen Ventilationshohlraum (11) und ein Lufteinlassende (12) und ein Luftauslassende (13) umfasst, die mit dem Ventilationshohlraum (11) in Verbindung stehen, wobei der Ventilationskörper (10) ferner ein ringförmiges Gehäuse (14) umfasst, das dazu konfiguriert ist, den Ventilationshohlraum (11) zu bilden, wobei das ringförmige Gehäuse (14) mit einem ringförmigen Hohlraum (15) im Inneren ausgebildet ist, ein Lufteinlass (16) und ein Luftauslass (17), die mit dem ringförmigen Hohlraum (15) in Verbindung stehen, an dem ringförmigen Gehäuse (14) angeordnet sind, der Luftauslass (17) mit dem ringförmigen Hohlraum (15) und dem Ventilationshohlraum (11) in Verbindung steht, der Luftauslass (17) eine Schlitzform hat, die sich entlang einer Umfangsrichtung des ringförmigen Gehäuses (14) erstreckt, und so angeordnet ist, dass er in der Lage ist, Gasströme nach außen zum Luftauslassende (13) zu leiten;
**dadurch gekennzeichnet, dass**
der Ventilator eine Atemmaske umfasst, die mit einem Atemhohlraum versehen ist, der Ventilationskörper (10) mit der Atemmaske verbunden ist und das Luftauslassende (13) abgedichtet ist und mit dem Atemhohlraum in Verbindung steht.

2. Ventilator nach Patentanspruch 1, wobei der Luftauslass (17) nahe dem Lufteinlassende (12) angeordnet ist; und/oder
eine Seitenkante (171) des Luftauslasses (17) nahe dem Lufteinlassende (12) so angeordnet ist, dass sie sich in Richtung der anderen Seitenkante (172) des Luftauslasses (17) biegt und erstreckt, und die Seitenkante (171) im Vergleich zu der anderen Seitenkante (172) näher an einer Mittelachse des ringförmigen Gehäuses (14) in einer radialen Richtung des ringförmigen Gehäuses (14) liegt.

3. Ventilator nach Patentanspruch 2, wobei,
die andere Seitenkante (172) des Luftauslasses (17) so angeordnet ist, dass sie sich in einer Richtung weg von der Seitenkante (171) biegt und erstreckt; und/oder
ein Mindestabstand zwischen der Seitenkante (171) des Luftauslasses (17) und der anderen Seitenkante (172) weniger als 2 mm beträgt.

4. Ventilator nach einem der Patentansprüche 2-3, wobei,
eine Breite des ringförmigen Hohlraums (15) entlang einer Richtung vom Lufteinlassende (12) zum Luftauslassende (13) allmählich abnimmt; und/oder
ein Durchmesser des Ventilationshohlraums (11) entlang einer Richtung vom Lufteinlassende (12) zum Luftauslassende (13) allmählich zunimmt.

5. Ventilator nach einem der Patentansprüche 1-4, wobei der Ventilator eine Basis (20) und einen Antriebsmotor (30) umfasst, die Basis (20) mit einem Hohlraum (21) im Inneren ausgebildet ist, ein Lufteinlassloch (22) und ein Luftauslassloch (23), die mit dem Hohlraum (21) in Verbindung stehen, an der Basis (20) angeordnet sind, der Antriebsmotor (30) im Inneren des Hohlraums (21) angeordnet ist, das ringförmige Gehäuse (14) auf der Basis (20) befestigt ist und der Lufteinlass (16) so ausgebildet ist, dass er abgedichtet ist und mit dem Luftauslassloch (23) in Verbindung steht.

6. Ventilator nach Patentanspruch 5, wobei,
der Ventilator ein Pufferteil (40) umfasst, wobei sich das Pufferteil (40) im Inneren des Hohlraums (21) befindet und so angeordnet ist, dass es den Antriebsmotor (30) umgibt; und/oder
die Basis (20) eine Bodenwand (24) und eine umgebende Wand (25) umfasst, die dazu konfiguriert sind, den Hohlraum (21) zu bilden, wobei eine obere Öffnung des Hohlraums (21) das Luftauslassloch (23) bildet und das Lufteinlassloch (22) an der umgebenden Wand (25) angeordnet ist.

7. Ventilator nach Patentanspruch 6, wobei der Antriebsmotor (30) säulenförmig ausgebildet ist, das Pufferteil (40) zylinderförmig ausgebildet und zwischen dem Antriebsmotor (30) und der umgebenden Wand (25) geklemmt ist, das Pufferteil (40) luftdurchlässig ist.

8. Ventilator nach einem der Patentansprüche 5-7, wobei der Ventilator eine Hauptsteuerkomponente umfasst, die Hauptsteuerkomponente in dem Hohlraum (21) angeordnet und dazu konfiguriert ist, den Betrieb des Antriebsmotors zu steuern, und mit einem Steuerterminal kommunikativ verbunden ist.

9. Ventilator nach einem der Patentansprüche 1-8, wobei der Ventilator einen Wärme-Feuchtigkeits-Austauscher umfasst, wobei der Wärme-Feuchtigkeits-Austauscher am Luftauslassende (13) installiert ist; und/oder
der Ventilator eine Filtervorrichtung umfasst, wobei die Filtervorrichtung am Lufteinlassende (12) angeordnet ist.

## Revendications

1. Ventilateur, le ventilateur comprenant un corps de ventilation (10), le corps de ventilation (10) comprenant une cavité de ventilation (11) et une extrémité d'entrée d'air (12) et une extrémité de sortie d'air (13) communiquant avec la cavité de ventilation (11), le corps de ventilation (10) comprenant en outre une coque annulaire (14) conçue pour former la cavité de ventilation (11), la coque annulaire (14) étant formée d'une cavité annulaire (15) à l'intérieur, une entrée d'air (16) et une sortie d'air (17) communiquant avec la cavité annulaire (15) étant disposées sur la coque annulaire (14), la sortie d'air (17) communiquant avec la cavité annulaire (15) et la cavité de ventilation (11), la sortie d'air (17) ayant une forme de fente s'étendant le long d'un sens circonférentiel de la coque annulaire (14) et étant disposée pour être capable de guider le gaz s'écoulant vers l'extérieur vers l'extrémité de sortie d'air (13) ;
**caractérisé en ce que**
le ventilateur comprend un masque de respiration pourvu d'une cavité de respiration, le corps de ventilation (10) étant raccordé au masque de respiration et l'extrémité de sortie d'air (13) étant fabriquée pour être hermétiquement scellée et communiquer avec la cavité de respiration.

2. Ventilateur selon la revendication 1, la sortie d'air (17) étant disposée près de l'extrémité d'entrée d'air (12) ; et/ou
un bord latéral (171) de la sortie d'air (17) près de l'extrémité d'entrée d'air (12) étant disposé pour fléchir et s'étendre vers l'autre bord latéral (172) de la sortie d'air (17), et le bord latéral (171) étant plus proche d'un axe central de la coque annulaire (14) dans un sens radial de la coque annulaire (14) comparé à l'autre bord latéral (172).

3. Ventilateur selon la revendication 2,
l'autre bord latéral (172) de la sortie d'air (17) étant disposé pour fléchir et s'étendre dans un sens opposé au bord latéral (171) ; et/ou
un espacement minimal entre le bord latéral (171) de la sortie d'air (17) et l'autre bord latéral (172) étant inférieur à 2 mm.

4. Ventilateur selon l'une quelconque des revendications 2 à 3,
une largeur de la cavité annulaire (15) baissant progressivement le long d'un sens depuis l'extrémité d'entrée d'air (12) vers l'extrémité de sortie d'air (13) ; et/ou
un diamètre de la cavité de ventilation (11) augmentant progressivement le long d'un sens depuis l'extrémité d'entrée d'air (12) vers l'extrémité de sortie d'air (13).

5. Ventilateur selon l'une quelconque des revendications 1 à 4, le ventilateur comprenant une base (20) et un moteur d'alimentation (30), la base (20) étant formée avec une cavité (21) à l'intérieur, un trou d'entrée d'air (22) et un trou de sortie d'air (23) communiquant avec la cavité (21) étant disposés sur la base (20), le moteur d'alimentation (30) étant disposé à l'intérieur de la cavité (21), la coque annulaire (14) étant installée sur la base (20) et l'entrée d'air (16) étant fabriquée pour être hermétiquement scellée et communiquer avec le trou de sortie d'air (23).

6. Ventilateur selon la revendication 5,
le ventilateur comprenant une partie tampon (40), la partie tampon (40) étant localisée à l'intérieur de la cavité (21) et disposée pour entourer le moteur d'alimentation (30) ; et/ou
la base (20) comprenant une paroi inférieure (24) et une paroi périphérique (25) conçues pour former la cavité (21), une ouverture supérieure de la cavité (21) formant le trou de sortie d'air (23), et le trou d'entrée d'air (22) étant disposé sur la paroi périphérique (25).

7. Ventilateur selon la revendication 6, le moteur d'alimentation (30) étant formé pour présenter une forme de colonne, la partie tampon (40) étant formée pour présenter une forme de cylindre et fixée entre le moteur d'alimentation (30) et la paroi périphérique (25), la partie tampon (40) étant perméable à l'air.

8. Ventilateur selon l'une quelconque des revendications 5 à 7, le ventilateur comprenant un composant de commande principal, le composant de commande principal étant disposé dans la cavité (21) et conçu pour commander le fonctionnement du moteur d'alimentation, et étant raccordé de manière à pouvoir communiquer à un terminal de commande.

9. Ventilateur selon l'une quelconque des revendications 1 à 8, le ventilateur comprenant un échangeur de chaleur-humidité, l'échangeur de chaleur-humidité étant installé au niveau de l'extrémité de sortie d'air (13) ; et/ou
le ventilateur comprenant un appareil de filtration, l'appareil de filtration étant disposé au niveau de l'extrémité d'entrée d'air (12).
